# EUROPEAN PATENT SPECIFICATION

(11) **EP 3 349 351 B1**
(45) Date of publication and mention of the grant of the patent: **17.11.2021**
(21) Application number: 16843659.0
(22) Date of filing: 08.09.2016
(51) Int. Cl.: H01L 31/0525, H01L 31/054, H02S 10/10, H01L 35/00, H02S 40/22, H02S 40/44

(54) **INTEGRATED SOLAR ENERGY UTILIZATION APPARATUS AND SYSTEM**
VORRICHTUNG UND SYSTEM FÜR INTEGRIERTE SOLARENERGIENUTZUNG
APPAREIL ET SYSTÈME D'UTILISATION D'ÉNERGIE SOLAIRE INTÉGRÉE

(30) Priority: 11.09.2015 CN 201510576621
(43) Date of publication of application: 18.07.2018
(73) Proprietor: Boly Media Communications (Shenzhen) Co., Ltd, Shenzhen, Guangdong 518055 (CN)
(72) Inventor: HU, Xiaoping, Shenzhen, Guangdong 518109 (CN)
(74) Representative: Manitz Finsterwald Patent- und Rechtsanwaltspartnerschaft mbB
(86) International application number: PCT/CN2016/098386
(87) International publication number: WO 2017/041721

(56) References cited:
- CN-A- 101 313 419
- CN-A- 101 673 780
- CN-A- 101 826 823
- CN-A- 102 104 346
- CN-A- 102 487 255
- GB-A- 2 321 338
- JP-B2- H 084 146
- US-A1- 2009 250 096
- US-A1- 2012 291 849
- CHÁVEZ-URBIOLA E A ET AL: "Solar hybrid systems with thermoelectric generators", SOLAR ENERGY, PERGAMON PRESS. OXFORD, GB, vol. 86, no. 1, 10 October 2011 (2011-10-10), pages 369-378, XP028350664, ISSN: 0038-092X, DOI: 10.1016/J.SOLENER.2011.10.020 [retrieved on 2011-10-25]
- P.OOSHAKSARAEI ET AL: "Terrestrial Applications of Bifacial Photovoltaic Solar Panels", PROCEEDINGS OF THE 10TH WSEAS INTERNATIONAL CONFERENCE ON SYSTEM SCIENCE AND SIMULATION IN ENGINEERING (ICOSSSE'11), 3 November 2011 (2011-11-03), pages 128-131, XP055575137, Penang, Malaysia ISBN: 978-1-61804-041-1
- DAVID BARLEV ET AL: "Innovation in concentrated solar power", SOLAR ENERGY MATERIALS AND SOLAR CELLS, ELSEVIER SCIENCE PUBLISHERS, AMSTERDAM, NL, vol. 95, no. 10, 12 May 2011 (2011-05-12), pages 2703-2725, XP028241464, ISSN: 0927-0248, DOI: 10.1016/J.SOLMAT.2011.05.020 [retrieved on 2011-06-16]
- SIMON RITTMANN ET AL: "Essential prerequisites for successful bioprocess development of biological CH 4 production from CO 2 and H 2", CRC CRITICAL REVIEWS IN BIOTECHNOLOGY, vol. 35, no. 2, 11 September 2013 (2013-09-11), pages 141-151, XP055578840, US ISSN: 0738-8551, DOI: 10.3109/07388551.2013.820685

## Description

### TECHNICAL FIELD

The present disclosure relates to clean energy, and in particular, to integrated solar energy utilization apparatus and systems for utilizing solar energy. The features of the preamble of the independent claim are known from document CHAVEZ-URBIOLA E A ET AL: "Solar hybrid systems with thermoelectric generators", SOLAR ENERGY, vol. 86 (2012) 369-378, no. 1, 11.11.2011. Related technologies are known from US 2012/291849 A1; GB 2 321 338 A; JP H08 4146 B2; US 2009/250096 A1; document P. Ooshaksaraei et al: "Terrestrial Applications of Bifacial Photovoltaic Solar Panels", Proc. of the 10th WSEAS ICOSSSE'11, 3.11.2011; document DAVID BARLEV ET AL: "Innovation in concentrated solar power", SOLAR ENERGY MATERIALS AND SOLAR CELLS, vol. 95, no. 10, 12 May 2012; document Simon Rittmann ET AL: "Essential prerequisites for successful bioprocess development of biological CH4 production from CO2 and H2", CRC CRITICAL REVIEWS IN BIOTECHNOLOGY, vol. 35, no. 2, 11 September 2013.

### PRIOR ART

With increasing emphasis on environmental protection, solar energy systems are growing in popularity. For current solar systems, they can be divided into two categories basically: power supply and heat supply.

Solar energy systems for power supply can be further grouped into systems that generate electricity by photovoltaic conversion and systems that generate electricity by photothermal conversion. Systems that generate electricity by photovoltaic conversion can be implemented by using photovoltaic panels to directly convert absorbed solar energy into electric energy; while systems that generate electricity by photothermal conversion can be implemented by using solar energy to heat working medium to vaporize and driving power generation equipment such as steam turbine by the vaporized medium for power generation. In addition, there are solar systems that generate electricity by utilizing thermal power directly; for example, solar systems that uses Stirling generators or other direct thermoelectric conversion devices.

However, no matter what type of solar power system is employed, current energy conversion efficiency still needs to be enhanced.

### SUMMARY

The present invention is defined in the independent claim. According to an example of the present disclosure not necessarily falling under the scope of the claims, there is provided an integrated solar energy utilization apparatus including at least one photovoltaic conversion member and at least one thermoelectric conversion member. The thermoelectric conversion member includes at least one first heat-conducting end for inflow of heat, and the photovoltaic conversion member is connected to the first heat-conducting end in a thermally conductive manner. The thermoelectric conversion member may be a temperature-difference power generation element or an energy-storing power generation element. When the thermoelectric conversion member is the temperature-difference power generation element, it further includes at least one second heat-conducting end for outflow of heat, and when the temperature of the first heat-conducting end is higher than that of the second heat-conducting end, the thermoelectric conversion member outputs electric energy. When the thermoelectric conversion member is the energy-storing power generation element, it further includes a working substance for directly storing heat flowing to the first heat-conducting end or converting the heat into chemical energy for storage, and converting the stored energy into electric energy for output. The direct storage of thermal energy includes the temperature rise or phase change of the working substance, such as liquefaction and vaporization. The chemical energy can include the stored energy of chemical changes of the molecules of the substance (such as ionization, electrolysis, compounding and decomposition, etc.).

According to an example not part of the present disclosure, there is provided a solar energy utilization system including the above integrated solar energy utilization apparatus and a first circulating arrangement. The first circulating arrangement is an open or closed circulating arrangement including a container for heating a first working fluid, a first pipeline network, at least one valve and at least one device node. The container is provided with at least one first working fluid inlet and at least one first outcome outlet, and at least the second heat-conducting end of the integrated solar energy utilization apparatus is connected in a thermally conductive manner to the first working fluid in the container. The first pipeline network is connected to at least the first outcome outlet or to at least the first working fluid inlet and the first outcome outlet. The valve is used for controlling the connection and disconnection of a section of pipelines within the first pipeline network. The device node is connected in the first pipeline network for storage, or for energy conversion, or for energy exchange.

In the integrated solar energy utilization apparatus according to the present disclosure, the photovoltaic conversion member and the thermoelectric conversion member are integrated together and the thermoelectric conversion member is used to further convert the heat generated by the photovoltaic conversion member into electricity, so that the the solar energy not utilized by the photovoltaic conversion member can be utilized again, thereby effectively enhancing the energy conversion rate for solar power generation.

The integrated solar energy utilization apparatus used as a heat source is introduced to the circulating system to heat the working fluid in the circulating system the according to the solar energy utilization system of the present disclosure, which can not only make full use of the sunlight energy but also expand more functions, for example, solar energy can be converted to other available energy by the circulating system, such as kinetic energy provided by steam or thermal energy or chemical energy for long-term storage, and the like.

Specific examples according to the present invention are described in detail below with reference to the accompanying drawings.

### BRIEF DESCRIPTION OF THE DRAWINGS

The examples depicted on Figs 1, 6-11 and 13-15 refer to example not part of the present invention, although the passages of the following description referring to these figures might label these examples as embodiments.

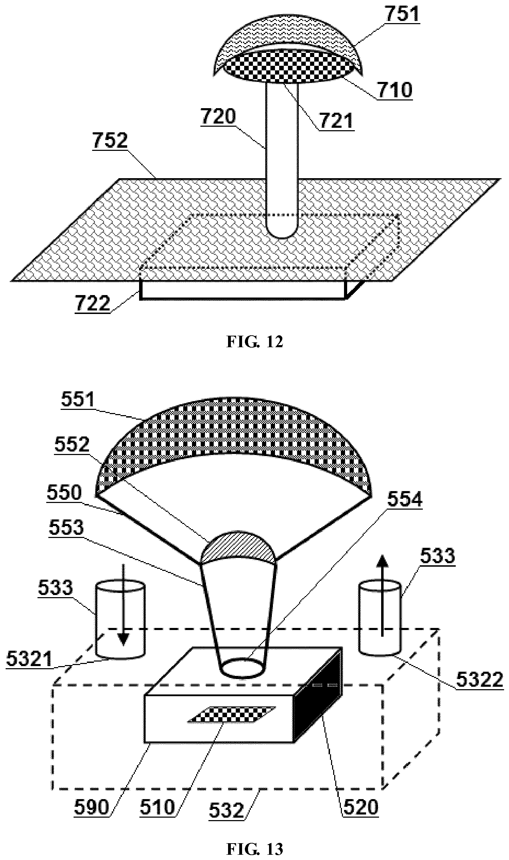

Fig. 12 depicts an embodiment according to the present invention.
FIG. 1 is a schematic structural diagram of an integrated solar energy utilization apparatus according to the present disclosure;
FIG. 2 is a schematic diagram of two coaxial surfaces for generating Fresnel refracting surfaces in the present disclosure;
FIG. 3 is a schematic diagram of a light-condensing member having two tooth surfaces in the present disclosure;
FIG. 4 is a schematic structural diagram of a reflective Fresnel lens in the present disclosure;
FIG. 5 is a schematic diagram of a connection in a thermally conductive manner among a plurality of thermoelectric conversion members according to the present disclosure;
FIG. 6 is a schematic structural diagram of a solar energy utilization system according to the present disclosure;
FIG. 7 is a schematic diagram of an integrated solar energy utilization apparatus of Embodiment 1;
FIG. 8 is a schematically equivalent circuit diagram of a thermoelectric conversion member of Embodiment 1;
FIG. 9 is a schematic diagram of a PCB integrated with a connection circuit and a heat-conducting member in Embodiment 1;
FIG. 10 is a schematic diagram of a switch arrangement of the connection circuit in Embodiment 1;
FIG. 11 is a schematically equivalent circuit diagram of the thermoelectric conversion member after circuit switching in Embodiment 1;
FIG. 12 is a schematic diagram of an integrated solar energy utilization apparatus of Embodiment 2;
FIG. 13 is a schematic diagram of a solar energy utilization system of Embodiment 3;
FIG. 14 is a schematic diagram of a solar energy utilization system of Embodiment 4;
FIG. 15 is a schematic diagram of a solar energy utilization system of Embodiment 5.

### DETAILED EMBODIMENTS

In the following description, the embodiments 1, 3, 4 and 5 are not part of the invention. They are examples not covered by the scope of the claims.

The embodiment 2 is an embodiment according to the invention.

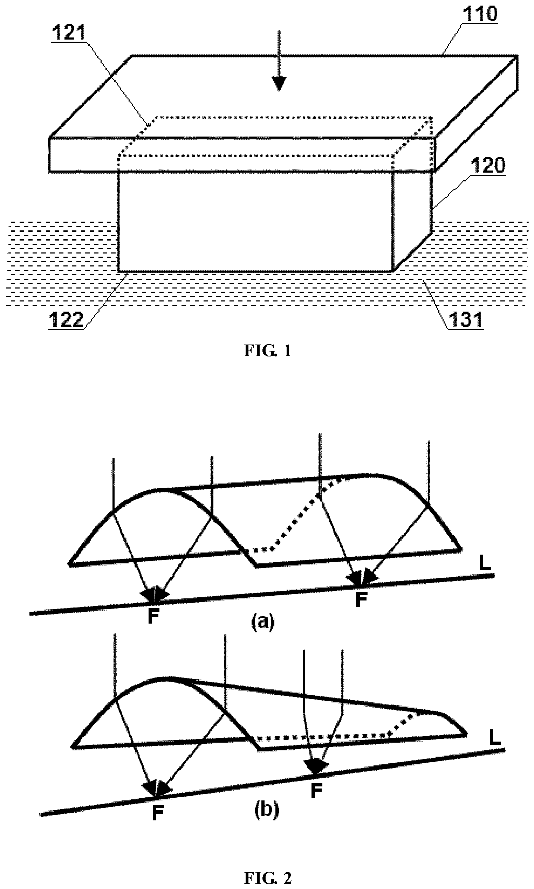

Referring to FIG. 1, a basic structure of the integrated solar energy utilization apparatus according to the present disclosure may include a photovoltaic conversion member 110 and a thermoelectric conversion member 120.

The photovoltaic conversion member 110 may be implemented by employing various elements capable of directly converting light energy into electric energy, such as photovoltaic panels, photovoltaic films, and the like. Said photovoltaic panels may be, for example, monocrystalline silicon photovoltaic panels, polycrystalline silicon photovoltaic panels, amorphous silicon photovoltaic panels, composite semiconductor material photovoltaic panel, or the like. The amount, type and arrangement of the photovoltaic conversion members in the apparatus may be designed as required, for example, in a series or parallel arrangement or a mixture thereof.

Preferably, at least one light-condensing member (not shown) may be further included in the apparatus for condensing sunlight to be provided to the photovoltaic conversion member. The light-condensing member may have at least one condensing and refracting surface which may have a large area to receive more sunlight. In addition, the condensing and refracting surface (or another surface of an optical element on which the condensing and refracting surface positioned) may be coated with a film for anti-reflection and enhancement of transmittance to reduce the reflection loss of sunlight in the light-condensing member and improve light-gathering efficiency. The anti-reflection coating film may also enhance corrosion resistance of the light-condensing member to some extent, thereby increasing the life of the light-condensing member. Moreover, an electric heating member such as a heating wire for snow removal or de-icing may be further provided on the lens of the light-condensing member so as to improve the adaptability of the apparatus to extreme environments.

It is particularly beneficial to integrate the condensing photovoltaic conversion member and the thermoelectric conversion member. In fact, the discovery of the thermoelectric conversion effect is much earlier than that of the photovoltaic conversion effect, and the usage of thermal energy precedes that of light energy for generating electricity. But the use of photovoltaic conversion is far more than the use of thermoelectric conversion at present. An important reason is that, only direct thermoelectric conversion can be used when the thermal energy is low, whereas the current photoelectric conversion efficiency far exceeds the direct thermoelectric conversion efficiency, and the cost of photovoltaic panels is much lower than that of thermoelectric converters at the same power output. Nevertheless, it is possible to reduce manyfold the use amount of the photovoltaic panels after condensing a large area of sunlight by using the light-condensing member, thereby making the generated heat energy more concentrated. In this case, only a small amount of thermoelectric conversion members are required to be able to greatly increase the direct power generation efficiency of the solar energy, greatly improving the cost performance of using the thermoelectric conversion members. When the intensity of heat energy is strong enough (that is, the condensed solar energy is strong enough), relatively high efficiency thermal power generation equipment, such as the well-known Stirling generators and steam generators, can be used to further improve the utilization efficiency of solar energy.

Preferably, the condensing and refracting surface can be a tooth surface provided by a Fresnel lens. Related concepts are described below for ease of understanding.

A Fresnel lens is a thin lens. The Fresnel lens is formed by dividing a continuous original curved surface of an ordinary lens into a number of segments, and placing the segments of curved surface on a same plane or same substantially smooth curved surface after the thickness of each segment is reduced. Such a discontinuous refractive surface evolved from the original curved surface can be called a Fresnel refractive surface, which is generally stepped or toothed. Theoretically, the Fresnel refractive surface has similar optical performance to the corresponding original curved surface, but has a greatly reduced thickness. A Fresnel refractive surface generated by an original curved surface (or part of the original curved surface) can be referred to as a Fresnel unit.

A traditional original curved surface for generating a Fresnel refractive surface is generally a curved surface symmetrical about an optical axis, such as a spherical surface, a paraboloid of revolution or other surface of revolution. Foci of the traditional original curved surface are located on one point, and thus called a "concurrent surface". In the present invention, the original curved surface can be a coaxial surface in any form, which can be specifically set as required in application. Said coaxial surface means a curved surface whose foci are located on a same straight line (but not necessarily on a same point), and the straight line is called a "coaxial line". A traditional concurrent surface can be regarded as a special example of a coaxial surface, the coaxial line of which regresses to a point. By using a coaxial but non-concurrent original curved surface, a sensing element to be provided at a focusing position can be expanded from a relatively small area (corresponding to the foci) to a strip (corresponding to the coaxial line composed of the foci), thereby improving signal collecting capability and being conducive to solving the problem of local overheat, without significantly increasing the cost. Typical coaxial surfaces include surfaces of revolution (including quadratic or high-order surfaces of revolution), columnar surfaces, tapered surfaces and the like. The columnar surfaces are also known as uniform-section coaxial surfaces. Regardless of the point where such a curved surface is cut along a direction perpendicular to its coaxial line, the shape and size of the resulting cross section are same. A cylindrical surface is a special example of a columnar surface. Cross sections of a tapered surface along its coaxial line have similar shapes but different sizes. A conical surface is a special example of a tapered surface. Fig. 2 shows the two types of coaxial surfaces described above, wherein a uniform-section coaxial surface is illustrated in Fig. 2(a), and a tapered coaxial surface is illustrated in Fig. 2(b), with foci F being located on their respective coaxial lines L.

A macroscopic refractive surface composed of one or more Fresnel units can be referred to as a toothed surface, and a substantially smooth or flat surface opposite thereto can be called a back surface. A toothed surface having only one Fresnel unit can be referred to as a "simple Fresnel refractive surface", and a toothed surface having two or more Fresnel units can be called a "composite Fresnel refractive surface". Generally, basic parameters (such as the area, the focal length, the shape of the corresponding original curved surface, and the number of concentric rings used for dividing the original curved surface) of the Fresnel units on the composite Fresnel refractive surface can be set flexibly to be all same, partially same or all different. In an embodiment, each Fresnel unit on the composite Fresnel refractive surface has its respective optical center, but their foci are located on a same point, or a same straight line, or a limited region. This can be achieved by spatial distribution of the Fresnel units forming the composite Fresnel refractive surface. It can be assumed that these Fresnel units are provided on a microscopic curved surface, such as a plane surface, quadratic surface (including a spherical surface, ellipsoidal surface, cylindrical surface, parabolic columnar surface, or hyperbolic columnar surface), a high-order polynomial curved surface (a general implementation of a non-spherical surface), or a bending surface or terraced surface formed by piecing a number of plane surfaces together.

The toothed surface and the back surface can be flexibly combined to form different types of elements. For example, a Fresnel lens with a toothed surface and a back surface can be referred to as a "simple Fresnel lens". Further, if the toothed surface is a "simple Fresnel refractive surface", such a lens is called a "single-side simple Fresnel lens"; and if the toothed surface is a "composite Fresnel refractive surface", such a lens is called a "single-side composite Fresnel lens". A Fresnel lens with toothed surfaces on both sides can be referred to as a "double-side Fresnel lens". Similarly, such lenses can be further classified into "double-side simple Fresnel lenses" and "double-side composite Fresnel lenses" according to the types of toothed surfaces. A double-side Fresnel lens, with one toothed surface being a simple Fresnel refractive surface, and the other toothed surface being a composite Fresnel refractive surface, can be referred to as a "double-side hybrid Fresnel lens". In addition, as a modification, if one of the toothed surfaces of a double-side Fresnel lens is a "simple Fresnel refractive surface", this toothed surface can be replaced by a traditional convex lens surface or convex lens surface.

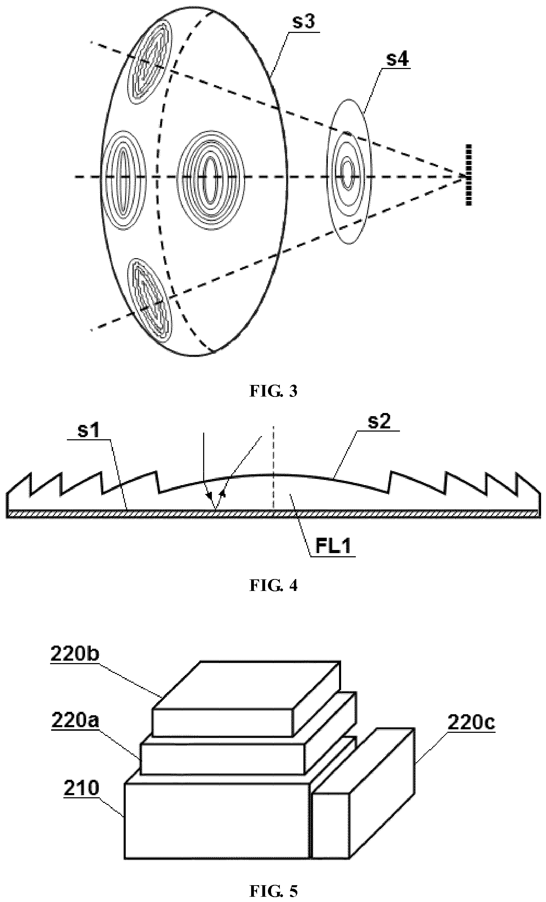

Providing two or more toothed surfaces on a same light path can ensure higher convergence capability of the converging system. Fig. 3 shows a converging system with two toothed surfaces, wherein a composite Fresnel refractive surface s3 and a simple Fresnel refractive surface s4 can be provided by one double-side Fresnel lens at the same time, and can also be provided by two single-side Fresnel lenses respectively.

It should be recognized that the two tooth sides of the system may be implemented by the same physical interface through arranging reflective surface. Referring to FIG. 4, the element FL1 may be provided with a reflective back side s1 (the inner surface is mirror). The back side s1 may be formed by, for example, plating a reflective film or bonding patches with reflective capability on the smooth surface of the single-sided Fresnel lens or other ways. Because of the reflection, the incident light path may pass through the physical refraction interface s2 twice. Therefore, such physical interface may equivalent to two tooth sides. The element FL1 may also be referred to as reflective double-sided Fresnel lens, and the concave and convex nature of the two tooth sides may be the same. By arranging the reflective back side, the number of the tooth sides in the light path may be simply increased, the production cost and installation cost may be reduced, and the use forms of the Fresnel lens may be greatly increased.

The light-condensing members and divergent lenses disclosed herein can be served as a Fresnel lens or combination thereof having a concave lens or optical characteristics of a concave lens. A Fresnel lens can be made of glass, resin, transparent plastic and other materials. When made of glass, the glass can also be tempered to enhance its strength and safety.

The thermoelectric conversion member 120 may include at least one first heat-conducting end 121 for inflow of heat; and the photovoltaic conversion member is connected in a thermally conductive manner to the first heat-conducting end 121. The conversion efficiency of the photovoltaic conversion member is generally not more than 20% currently, and large amount of thermal energy is generated at the same time. According to the present disclosure, the thermal energy can be conducted to the thermoelectric conversion member for further utilization.

In some embodiments, the first heat-conducting end may directly be contacted with the photovoltaic conversion member for heat conduction, for example, it may be generally disposed at the surface opposite to the surface of the photovoltaic conversion member illuminated by sunlight (as indicated by an arrow in the figure). In other embodiments, the first heat-conducting end may also indirectly exchange heat with the photovoltaic conversion member via a heat-conducting medium (such as heat-conducting silicone or heat-conducting liquid) or a heat-conducting member (such as a metal plate).

The thermoelectric conversion member may be implemented by employing various elements capable of converting thermal energy into electrical energy, such as a temperature-difference power generation element or an energy-storing power generation element. Referring to FIG.1, when the thermoelectric conversion member is a temperature-difference power generation element, it may further include at least one second heat-conducting end 122 for outflow of heat, and when the temperature of the first heat-conducting end is higher than that of the second heat-conducting end, the temperature-difference power generation element outputs electricity. The temperature-difference power generation element may specifically be, for example, a semiconductor thermoelectric converter, an Alkali-metal thermal electric converter, or the like. When the thermoelectric conversion member is an energy-storing power generation element, it may further include working substances, such as positive and negative electrodes, and an electrolyte, for converting the heat flowing to the first heat-conducting end into chemical energy for storage, and converting the stored chemical energy into electric energy for output. The energy-storing power generation element may specifically be, for example, a melt salt battery regenerated by heated. Heat generated by high temperatures can also be used to drive Stirling generators, steam generators, etc., resulting in power conversion efficiencies higher than using photovoltaic panels. It should be noted that the melt salt battery may also be provided in the apparatus as an energy-storing member for storing thermal energy or electric energy so that the apparatus may be able to storage energy for a long period. This not only overcomes the problems of instable solar power supply and unnecessary waste of energy, but also improves the utilization efficiency of solar energy.

Thermal energy has proven to be a form of energy that can be conserved over a long period (such as six months) and at high efficiencies (such as 99%). Thermal energy can be stored in high density by materials of high heat capacity (such as paraffin, nitrate) and their phase transitions. The stored thermal energy can generate electricity by directly using a thermoelectric conversion equipment such as a thermal battery (such as a melt salt battery), or it may be used to drive Stirling generators or steam generators with waste heat used as heat source of a hot water or heating system.

Making full use of heat generated by solar energy is therefore a very important and effective method to increase solar energy utilization and reduce the cost of a solar energy system. However, the current solar power generation system fails to pay enough attention to heat energy. One of important reasons is that the existing solar condensing system is too costly and inefficient. Whereas, the present disclosure preferably uses a Fresnel lens, in particular, a Fresnel lens condensing system having at least two condensing and refracting surfaces and/or a plurality of Fresnel lens units so as to reduce the cost of a condensing system while improving the light-gathering efficiency, allowing a more convenient and effective way to utilize the thermal energy generated by the solar energy. Furthermore, the present disclosure is different from an existing solar thermal power system (such as a Stirling solar generator) in that the system according to the present disclosure first receives solar energy with a photovoltaic conversion member to directly convert 20% or more of the solar energy into electric energy, and stores and utilizes the solar thermal energy which is not converted into electric energy (around 70%) by a thermoelectric conversion member and a further storage or utilization device, thereby greatly increasing the utilization efficiency of solar energy while solving the problems of long-term energy storage and unstable power generation.

The number, type and arrangement of the thermoelectric conversion members in the apparatus can be designed as required. When two or more thermoelectric conversion members are used, they may be of the same type or different types, and may be connected in a thermally conductive manner with the photovoltaic conversion member in series or in parallel. Referring to FIG. 5, three thermoelectric conversion members 220a, 220b, 220c are provided around a heat source (a photovoltaic conversion member or a heat-conducting member that conducts thermal energy thereto) 210. The members 220a and 220b are connected in series with the heat source in a thermally conductive manner. The so-called series connection refers to connecting the thermal energy outflow end of a previous member to the thermal energy inflow end of a latter member. The member 220c and the member 220a/220b are connected in parallel in a thermally conductive manner. The so-called parallel connection means that the members are connected to the same heat source independently of each other. In some embodiments, the thermoelectric conversion members 220a and 220c that are closest to the heat source may be of a type that operates at a higher temperature, such as an Alkali-metal thermal electric converter, a melt salt battery, a steam generator, or a Stirling thermal energy generator, and the thermoelectric conversion members 220b which is relatively far away from the heat source can be of a type that operates at a lower temperature, such as a semiconductor thermoelectric converter.

In addition, when a temperature-difference power generation element is employed, the second heat-conducting end may be connected in a thermally conductive manner with a cooling medium 131 to maintain the temperature difference for improving the conversion efficiency. For example, the second heat-conducting end (or heat-conducting member that conducts thermal energy thereto) may be disposed at the bottom of the apparatus and soaked in a. The liquid pool may specifically be a water tank, a pond, a lake, a river or an ocean. The outer surface of the bottom of the apparatus can be watertight or anticorrosive accordingly, and the design of the apparatus can also be adaptively designed, for example in the shape of a basin, for installation on water surface. The cooling medium used preferably also serves as a working substance for thermal energy store in order to facilitate further energy recovery, for example for providing hot water or room heating.

Preferably, at least one of the photovoltaic conversion member and the thermoelectric conversion member is disposed in an enclosed cavity, or both are disposed respectively in two enclosed cavities that are nested with each other, and the internal cavity may be preferably used for providing with photovoltaic conversion member. When the photovoltaic conversion member is arranged in the enclosed cavity, the enclosed cavity is provided with at least one light entrance through which the sunlight can access into the enclosed cavity by guided by a light-guiding member.

Additional configuration or following additional elements (not shown) further integrated in the apparatus can also be included so as to better store and utilize the electric energy converted by the solar energy:
an electric-energy storage, which is in electrical connection with a power generation element (e.g. the photovoltaic conversion member and the thermoelectric conversion member) for storing electric energy, wherein the electric-energy storage can be selected from super capacitors, rechargeable batteries, melt salt batteries and air compressors; and the electric-energy storage can provide direct current output of various voltages directly through a connected DC voltage output device;
an AC inverter, which is in electrical connection with the electric-energy storage or with the power generation element directly for converting the direct current output by the power generation element into alternating current, for example 120V at 60Hz or 220V at 50Hz, wherein the AC inverter can not only provide AC output to a user directly, but also return electric energy to the power grid by an external network switchgear;
a status sensor and a status display for sensing and displaying various operating parameters, respectively, selected from one or more of the following: voltage, current, power, inventory of cooling medium or other working substances, pressure, temperature and etc.; and
a controller for controlling the working status of the apparatus based on the sensing result of the status sensor; for example, controlling start-up and shut-down of the entire apparatus, choosing an operating mode, and automatically raising an alarm when an abnormal parameter is sensed.

The integrated solar energy utilization apparatus according to the present disclosure can not only increase the efficiency of direct conversion of sunlight into electric energy significantly, but also reduce the temperature of the photovoltaic conversion member and accordingly prolongs its service life.

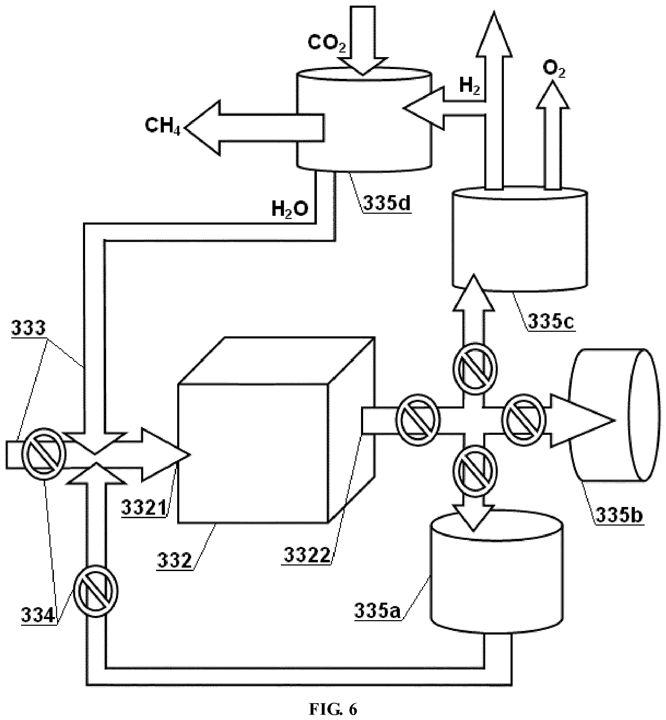

Referring to FIG. 6, a basic structure of a solar energy utilization system according to the present disclosure may include various integrated solar energy utilization apparatus according to the present disclosure and a first circulating arrangement.

The first circulating arrangement may be an open or closed circulating arrangement. As used herein, "open" means that working fluids in the system is open-cycle, that is, the working fluids can flow out of the system and be replenished from outside. For example, a system which receives cold water from an external water supply system and provides the user with heated water may be an open circulating arrangement when using water as the working fluid; or a system which electrolyzes water to generate hydrogen and oxygen and releases them may also be an open circulating arrangement. Pipelines in an open circulating arrangement do not need to be a loop. The so-called "closed" means that the working fluids in the system is closed-cycle, that is, the working fluids can be circulated within the system without any loss. For example, a system which compresses refrigerant used as working fluid and radiates heat therefrom inside a compressor and conducts an endothermic process of heat expansion at a heat exchanger may be a closed circulating arrangement. Pipelines in a closed circulating arrangement need to be a loop. A circulating arrangement can also be switched between open and closed; for example in the case of pipelines forming a loop, an open system may be changed to a closed system by closing valves of the system connected externally.

The first circulating arrangement may include a container 332, a first pipeline network consisting of several pipelines 333 (not fully shown, arrows in the figure presenting the flow of fluid in the pipelines), several valves 334 (not fully shown) and several device nodes 335a, 335b, 335c, 335d.

The container 332 is used to heat a first working fluid. The container is provided with at least one first working fluid inlet 3321 and at least one first outcome outlet 3322. At least the second heat-conducting end of the integrated solar energy utilization apparatus (not shown) is connected in a thermally conductive manner to the first working fluid in the container. For example, the second heat-conducting end may be immersed in the container.

In some embodiments, the integrated solar energy utilization apparatus may be wholly or partially immersed in the container. In other embodiments, the container may also be a part of the integrated solar energy utilization apparatus through which the first working medium flows. For the open circulating arrangement, the first working fluid can be water or air. For the closed circulating arrangement, the first working fluid can be other types of material as needed, such as alcohol, refrigerant (freon, etc.), liquid nitrogen.

The first pipeline network is connected to at least the first outcome outlet or, with reference to FIG. 3, at least to the first working fluid inlet and the first outcome outlet.

Valves are used to control the connection and disconnection of the pipelines in the first pipeline network, and each valve controls a section of pipelines in this embodiment. Valves may be configured to all or part of the pipelines as required. For the sake of simplicity, some of the valves provided in the pipelines are omitted in the figure. The valve can be an automatic valve, such as a valve that is opened or closed by pressure in the pipeline, or a valve that is electrically controlled in accordance with the instructions of a control system. The valves used may also be able to control flow.

Device nodes are connected in the first pipeline network for storage, or for energy conversion, or for energy exchange. Rich functionality can be achieved by configuring various device nodes.

In FIG. 6, the first working fluid is assumed to be freshwater or seawater and a first outcome water vapor. The device node 335a is a turbine generator that can generate steam using steam. The device node 335b is a steam storage tank or an air compressor that can be used to store energy. The device nodes 335c, 335d are an electrolytic furnace and a reacting furnace, respectively, which are sequentially connected according to the flow of the outcome. The electrolytic furnace is used for receiving water vapor and electrolyzing it to generate hydrogen (H₂) and oxygen (O₂). Oxygen generated by electrolysis can be injected into a gas storage tank (not shown) to form an industrial or medical oxygen cylinder. Hydrogen generated by electrolysis can be compressed by a compressor and stored to be used as a fuel for a hydrogen fuel cell or an internal combustion engine and alternatively or alternatively, be used to generate further storable energy sources. The reacting furnace is used to receive the hydrogen generated by the electrolytic furnace and the gas containing carbon dioxide (CO₂) from the outside (for example, air) and react to form methane (CH₄) and water (H₂O). The reacting furnace needs to be heated. For example, a way of condensing solar energy to heat may be adopted to obtain high temperature required for reaction; and in some embodiments, a way of fully electrical heating or auxiliary electrical heating may be adopted. Methane produced by the reacting furnace can be compressed and stored, or can be compressed and stored after passing through a safe (spark-free) turbine generator to achieve the dual effects of power generation and cooling. Water produced by the reacting furnace can be returned to the container 332 for circulation of water and reuse of waste heat.

In some embodiments, one or several different device nodes may be selected. In other embodiments, a plurality of device nodes of a sort may be provided. For example, a plurality of vapor storage devices and a turbine generator may be arranged in series or in parallel according to the amount of steam generated. By deploying a variety of device nodes, not only can solar energy be fully utilized, but also short-term or long-term energy storage, desalination, and industrial oxygen production can be achieved.

The apparatus and systems according to the present disclosure are exemplified below.

### Embodiment 1

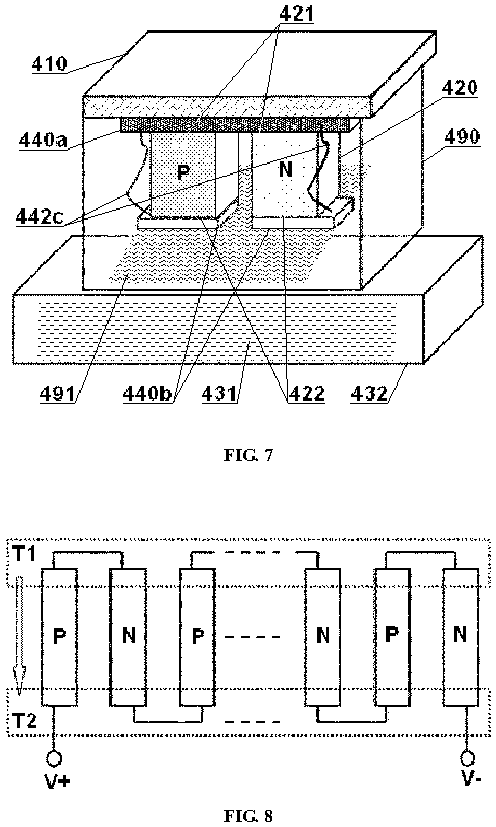

Referring to FIG. 7 to FIG. 11, an embodiment of the integrated solar energy utilization apparatus according to the present disclosure may include a photovoltaic conversion member 410 and a thermoelectric conversion member 420.

In this embodiment, a photovoltaic panel, which may consist of a plurality of photovoltaic modules, is employed as the photovoltaic conversion member; and a semiconductor thermoelectric converter, which may be formed by several pairs of P-type and N-type electrical arms connected in series or in parallel or in a hybrid type, is employed as the thermoelectric conversion member. A connection circuit may be provided for the thermoelectric conversion member according to design requirements thereof. For example, a first connection circuit may be configured to provide a conductive connection between the components at the first heat-conducting end of the thermoelectric conversion member, or to provide a conductive connection between the photovoltaic conversion member and the thermoelectric conversion member. A second connection circuit may also be configured to provide a conductive connection between the components at the second heat-conducting end of the thermoelectric conversion member. Of course, if the thermoelectric conversion member selected does not have the second heat-conducting end, it may not be necessary to provide the second connection circuit.

Referring to Fig. 8, there shows an equivalent circuit diagram of the semiconductor thermoelectric converter. When a temperature T1 is larger than a temperature T2 (T1 > T2), the heat flows in the direction of the arrow to output the electric energy between the positive electrode V + and the negative electrode V-. Each electrical arm has a first heat-conducting end 421 and a second heat-conducting end 422. For the sake of simplicity, only a pair of P-type and N-type arms is shown in FIG. 7.

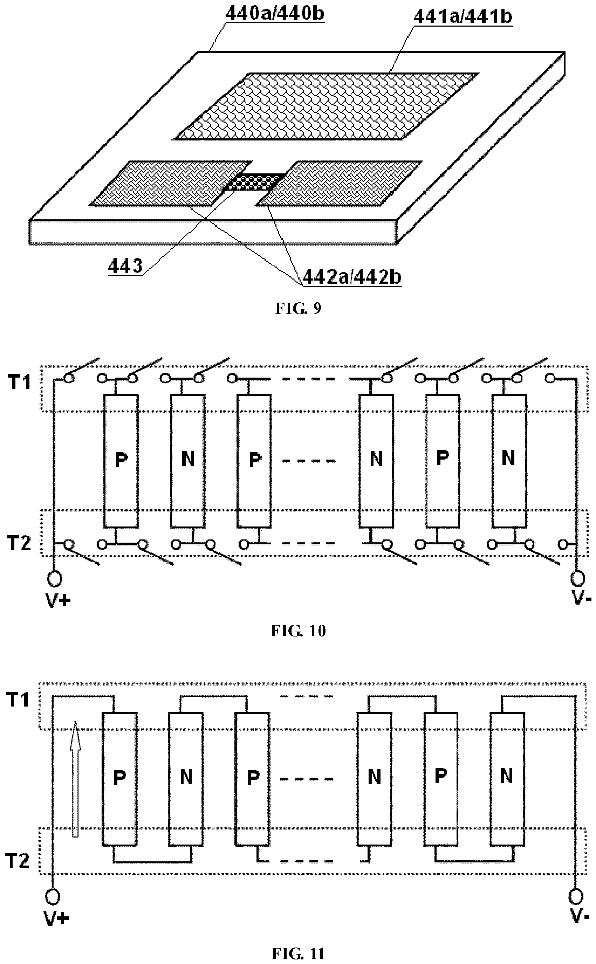

In this embodiment, the photovoltaic panel 410 is connected in a thermally conductive manner to the first heat-conducting end 421 through a first heat-conducting member 441a. Also, referring to FIG. 9, as a preferred embodiment, the first heat-conducting member and the first connection circuit 442a are integrated on the same substrate 440a. The substrate may be rigid or flexible; for example, a printed circuit board PCB or a flexible printed circuit board FPC may be used. It should be noted that only a simple integration of the circuit and the heat-conducting member, i.e. being arranged at different regions of the substrate separately from each other, is shown in FIG. 9. However, in other embodiments, if the substrate itself has good thermal conductivity, for example the substrate is made of metal and cladded with an insulating material, the substrate itself can be regarded as a heat-conducting member.

Similarly, at least one second heat-conducting member 441b may further be included for conducting heat from the second heat-conducting end. Likewise, the second heat-conducting member may also be integrated with the second connecting circuit 442b on the same substrate 440b. Also, if an electrical connection is required between the first connection circuit and the second connection circuit, an additional connection line 442c may be provided between the two substrates.

To adapt to some conditions of use, the apparatus may also include an enclosed cavity 490 (which is transparent in the figure for the purposes of illustration, but it may actually be transparent or opaque), and the photovoltaic panel is arranged on an outer surface of the enclosed cavity, for example the photovoltaic panel may be formed as part of the wall of the cavity. The thermoelectric conversion member is arranged within the cavity, and the second heat-conducting end exchanges heat with external environment through another outer surface of the cavity. For example, the second heat-conducting end or the heat-conducting member may be immersed in a non-conductive, thermally-conductive liquid 491 for heat exchange with the external environment. In order to further utilize the thermal energy not converted by the thermoelectric conversion member, the enclosed cavity 490 may also be arranged in a water tank 432 to heat the water 431 therein so as to provide hot water by using waste heat (water supply pipes is not shown in the figure). Of course, the water tank 432 may also be a reservoir, a lake or an ocean.

It is particularly beneficial to integrate the connection circuit and the heat-conducting member together by using PCB. On the one hand, the integrated design guarantees the stability and reliability of the thermal and electrical paths of the apparatus. On the other hand, it also simplifies and compacts the structure, making for better conduction of heat. More importantly, based on existing rich and mature PCB circuit design and production technology, it is easy to design a variety of single-layer or multi-layer circuits on the substrate, and integrate various measurement, control, switching devices, such as a switch 443 in FIG. 9 to achieve desired functions.

Referring to FIG. 10, an exemplary switch arrangement is shown, in which the first connection circuit and the second connection circuit are conductively connected, and a plurality of switches are provided in the whole circuit. Obviously, by controlling the on-off of the switch, the equivalent circuit shown in FIG. 8 can be easily obtained so that the semiconductor thermoelectric converter operates in a normal mode, i.e. T1> T2, and outputs the electric energy.

When it is necessary to use the second heat-conducting end as a working face for heating, the positive electrode V+ and the negative electrode V- may be connected to a switch (not shown) connected with an external power supply to input electric energy to the thermoelectric conversion member shown in FIG. 8 based on the functional reversibility of the thermoelectric conversion member, so that the temperature T1 of the first heat-conducting end decreases and the temperature T2 of the second heat-conducting end increases. The external power supply may be, for example, a photovoltaic conversion member 410 or other external power source.

The switches shown in FIG. 10 can also be used for circuit switching and obtain an equivalent circuit shown in FIG. 11 for example, which is suitable for a case where the temperatures of the first heat-conducting end and the second heat-conducting end are reversed. In such case, the second heat-conducting end is also used for inflow of heat, and the first heat-conducting end is also used for outflow of heat. When the temperature T2 of the second heat-conducting end is higher than the temperature T1 of the first heat-conducting end, the thermoelectric conversion member outputs electric energy. An applicable scene for this reverse power generation mode is that: hot water 431 heating during daylight which is not exhausted at night can be switched to such mode and power generation is continued by the heat of the hot water. Another applicable scene is that: the enclosed cavity 490 is immersed in seawater or lake water in a colder area; and the apparatus operates in a normal mode in daylight when the temperature of the photovoltaic panel is higher than water temperature, while the apparatus is switched to be operated in the reverse power generation mode to continue generating electricity at night when the water temperature is higher than the temperature of the photovoltaic panel.

Of coursc, the thermoelectric conversion member shown in FIG. 11 may also be input with electric energy as required, allowing the temperature of the second heat-conducting end to be decreased and the temperature of the first heat-conducting end to be increased, which for example can heat the photovoltaic panel for de-icing or defrosting.

The apparatus of the embodiment is suitable for being installed on the surface of water when used for building large-scale solar power plants. Because solar energy systems usually need to occupy a large area, installation on the surface of the water can effectively save land occupied, and can easily cool the apparatus, which helps to improve energy conversion efficiency and service life.

Thought only the semiconductor thermoelectric converter is taken as an example for description in this embodiment, those skilled in the art may understand that other temperature-difference thermoelectric conversion members can be employed, and specific circuit designs and switch control methods may be determined according to the types of actual components and application contexts as required.

### Embodiment 2

Referring to FIG. 12, another embodiment of the integrated solar energy utilization apparatus according to the present disclosure may include a photovoltaic conversion member 710, a thermoelectric conversion member 720 and a light-condensing member served as a transmission-type Fresnel lens 751 and a reflective Fresnel lens 752.

The photovoltaic conversation member 710 employs a double-sided element capable of absorbing incident sunlight from both front and back sides, for example a double-sided double-layered photovoltaic panel, arranged between the transmission-type Fresnel lens and the reflective Fresnel lens, so that both sides could receive the sunlight condensed by its facing lens respectively.

The thermoelectric conversion member 720 is formed as a support of the photovoltaic conversation member, with its first heat-conducting end 721 fixed with respect to the photovoltaic conversation member and its second heat-conducting end 722 fixed with respect to the reflective Fresnel lens. Referring to FIG. 12, for example, the second heat-conducting end can be arranged under the reflective Fresnel lens. In other embodiments, the second heat-conducting end may also be connected in a thermally conductive manner to the reflective Fresnel lens. The reflective surface of the reflective Fresnel lens is usually coated with metal, providing good thermal conductivity. The coating of the reflective surface can be made of non-conductive material with thermal conductivity, such as alumina, etc. for security purposes.

The first hcat-conducting end and the second hcat-conducting end in the apparatus according to the present disclosure can employ the integrated substrate described in Embodiment 1 to configure the control circuit such as the switch and the switching operation mode to be suitable for different environments.

Due to the light-condensing member which enhances condensing ability to reduce the area of the photovoltaic panel and the thermoelectric conversion member which acts as a support of the photovoltaic panel, the apparatus in the embodiment is simple in structure and cost-effective in large-scale use, especially suitable for surface installation. If the thermoelectric conversion member has a function of switching circuits as the temperature-difference direction is reversed, the thermoelectric conversion member can generate electric power when the water surface temperature is higher than the atmospheric temperature.

The apparatus of this embodiment is also suitable for use as part of street lights or for installation in a parking lot.

### Embodiment 3

Referring to FIG. 13, an embodiment of a solar energy utilization system according to the present disclosure may include an integrated solar energy utilization apparatus and a first circulating arrangement.

The integrated solar energy utilization apparatus may include a photovoltaic conversion member 510, a thermoelectric conversion member 520, an enclosed cavity 590, a light-condensing member 550 and a light-guiding member 553.

The enclosed cavity 590 is provided with a light entrance. The term "enclosed" herein means that the sunlight incident on the cavity and/or a substance (if any) would not be freely dissipated. On the one hand, the sunlight entering the cavity through the light entrance does not dissipate even if it goes through the walls of the cavity and gets out of the cavity; for example, the cavity can be made of reflective material. On the other hand, the substance present in the cavity is completely enclosed in the cavity or communicated with the outside in a controlled manner; for example the substance can communicate with outside by means of a pipeline with a valve, which is also construed as "enclosed" due to controllability of such communication.

The photovoltaic conversion member 510, for example, a photovoltaic panel, is arranged on the inner wall of the enclosed cavity or in the inner space of the enclosed cavity. As a preferred embodiment, a light-reflecting member may be arranged on the inner surface of the enclosed cavity. For example, the inner surface of the enclosed cavity may be covered by photovoltaic panels and reflecting mirrors so that the sunlight entering the cavity can be utilized sufficiently by the photovoltaic conversion member.

The thermoelectric conversion member 520 is arranged at the outer wall of the enclosed cavity; in particular, its first heat-conducting end tightly surrounds the outside of the enclosed cavity so as to sufficiently absorb the heat generated in the enclosed cavity. In other embodiments, first heat-conducting end of the thermoelectric conversion member may at least partially surround the enclosed cavity.

The light-condensing member 550 is used for condensing the sunlight to the light entrance of the light-guiding member. In this embodiment, two condensing and refracting surfaces 551, 552 are sequentially arranged along the optical path to obtain a stronger convergence effect.

The light-guiding member 553 is tightly matched with its corresponding light entrance for guiding sunlight collected by the light-condensing member to enter the enclosed cavity through the light entrance. The light-guiding member can be made based on various light transmission techniques; for example, it can be made of a solid transparent material or a hollow pipe and coated with a reflective film on the outer surface or the inner surface thereof, so that the sunlight can only go to the light entrance and enter the cavity after entering the light-guiding member.

Preferably, in this embodiment, a divergent lens 554 (e.g. a concave Fresnel lens) may further be arranged at the light entrance. The divergent lens may allow the sunlight entering the light entrance to be diverged so as to avoid as much as possible the sunlight being reflected back to the wall (e.g. photovoltaic panels or mirrors) facing the light entrance and going out of the cavity through the light entrance. In other embodiments, the divergent lens can be replaced by other means to reduce or prevent the sunlight from being dissipated at the light entrance. For example, the light-guiding member may be inserted into the cavity to a suitable depth, or the exit light direction of the light-guiding member may have an angle with the normal direction of its facing inner wall, or a scatter element may be arrange at a position facing against the exit light direction of the light-guiding member within the cavity. Such different ways can be used together.

In this embodiment, one light entrance, and one corresponding light-guiding member and one light-condensing member are shown schematically. In other embodiments, a plurality of light entrances and a corresponding plurality of light-guiding members and a plurality of light-condensing members may also be provided in the enclosed cavity.

Similar to the description herein before, the first circulating arrangement may include a container 532, a first pipeline network consisting of several pipelines 533, several valves (not shown) and several device nodes (not shown). At least one first working fluid inlet 5321 and at least one first outcome outlet 5322 are provided on the container 532. In this embodiment, the integrated solar energy utilization apparatus is completely accommodated in the container 532, the working fluid of the first circulating system is water and the first outcome is water vapor.

In operation, cold water is gotten into the container 532 through the inlet 5321 in the direction of the arrow, evaporated with heat and then flowed out through the outlet 5322 in the direction of the arrow and is stored or utilized in the subsequent device nodes.

In other embodiments, the container 532 may also be replaced by the enclosed cavity 590.

With the enclosed solar energy utilization apparatus of this embodiment, on the one hand, the energy of the sunlight converged in the enclosed cavity is almost completely converted into thermal energy or electric energy, avoiding energy dissipation; on the other hand, the thermal energy converted by the conversion member is also utilized by heating the working fluid to further enhance the utilization efficiency of solar energy and to obtain richer functions such as hot water supply, desalination of sea water, re-generation of electricity by using water vapor and the like. In addition, due to the photovoltaic panels and mirrors and other components closed in the cavity, neither light pollution nor adverse impact on surrounding environment will be produced.

There are three obvious differences between the present embodiment and other solar power generation systems or solar thermal energy utilization systems. Firstly, solar energy is at first used for direct conversion into electric energy, and solar energy that is not converted into electric energy is converted into thermal energy and then reused. Secondly, a thermoelectric conversion member is arranged on a thermal path (i.e., the wall of the enclosed cavity 590 in the present embodiment) that conducts thermal energy to the working fluid. In other words, before the thermal energy is used to heat the working fluid, it is partially converted directly into electrical energy. Thirdly, both the photovoltaic conversion member and the thermoelectric conversion member are arranged in a relatively enclosed cavity or container so that both the light energy and the heat energy can be fully utilized.

### Embodiment 4

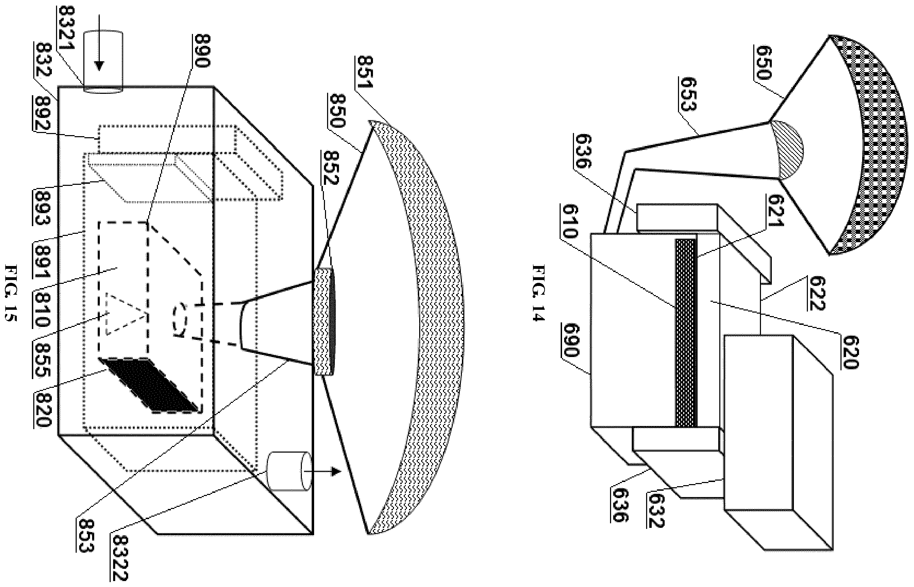

Referring to FIG. 14, another embodiment of a solar energy utilization system according to the present disclosure may include an integrated solar energy utilization apparatus and a first circulating arrangement.

The integrated solar energy utilization apparatus in the system of this embodiment which is similar to that in Embodiment 3 may include a photovoltaic conversion member 610, a thermoelectric conversion member 620, an enclosed cavity 690, a light-condensing member 650 and a light-guiding member 653. Except those special features described explicitly below, the aforesaid members may be described with reference to Embodiment 3. A heat storage material with good transparency and large heat capacity, such as paraffin, silica gel, transparent liquid and the like, may also be provided in the enclosed cavity 690.

The photovoltaic conversion member 510 is a photovoltaic panel arranged on the inner surface of the top of the enclosed cavity. The first heat-conducting end 621 of the thermoelectric conversion member 620 clings to the outer surface of the top of the enclosed cavity. A plane formed by the second heat-conducting end 622 of the thermoelectric conversion member can be used as a working face. The thermoelectric conversion member in this embodiment is a functionally reversible temperature-difference conversion element which can both utilize temperature difference for power generation and external power supply for cooling/heating.

For the sake of simplicity, only the container 632 (which may also be a heat-conducting member for conducting thermal energy for the container) of the first circulating system is shown in FIG. 14. Detailed illustrations for the rest may refer to the aforementioned description herein. The container 632 may be a mobile hot water tank.

When the working face is not in use, the hot water tank 632 may be placed on the working face to absorb the heat that is not converted by the member620. When the working face needs to be used, for example, when the working face is used for cooking as a heating surface, the hot water tank 632 may be removed for operation. In case of insufficient sunlight or the temperature of the working face failing to meet operating requirement, the function of the thermoelectric conversion member can also be reversed, that is, the electric energy is input to the thermoelectric conversion member to heat the working face. In some embodiments it is also possible to provide an electrically heated circuit directly on the working face, making it an electric furnace, if required.

In addition, a heat shield 636 (or an additional thermoelectric conversion member) may be provided on the outer surface of the enclosed cavity for heat preservation and safety protection. Thought only two heat shields (or an additional thermoelectric conversion member) are exemplarily shown, shields used to enclose the periphery of the enclosed cavity may be allowed in actual use.

With the system of this embodiment, the efficiency of using light energy directly for power generation is enhanced, and the waste heat which is not fully converted is also utilized fully. Such system is especially suitable for cold northern and field work.

### Embodiment 5

Referring to FIG. 15, there shows a solar energy utilization system according to another embodiment of the present disclosure. The solar energy utilization system in this embodiment, which is of high efficiency and high energy density and has functions of energy storage and heat supply, is especially suitable for places such as hotels, factories, office buildings, detached houses, power plants and the like.

The system of this embodiment includes an integrated solar energy utilization apparatus and a first circulating system. The integrated solar energy utilization apparatus includes a photovoltaic conversion member 810, a thermoelectric conversion member 820, an enclosed cavity 890, a light-condensing member 850, a light-guiding member 853 and a thermal-energy storage 891.

The enclosed cavity 890 is covered with photovoltaic panels and reflecting mirrors on the inner surface thereof and the thermoelectric conversion member 820 on the outer surface thereof. A conical reflector 855, which is positioned rightly facing the light entrance, may be provided in the enclosed cavity for changing the direction of the incident light so as to prevent it from being reflected from the light entrance. Solar energy is introduced into the interior of the enclosed cavity through the light-condensing member 850 consisting of two 851, 852 and the light-guiding member 853. The Fresnel condensing lenses used can be simple or combined-type Fresnel lenses.

The thermal-energy storage 891 is wrapped around the enclosed cavity, and is filled with a heat storage material having a large heat capacity, such as molten salt, paraffin, metallic compound with a low melting point, and the like. Thermal energy inside the thermal-energy storage can be connected in a thermally conductive manner to the outside via a heat flux-controlled heat exchange channel.

The heat flux-controlled heat exchange channel in this embodiment is exemplarily a removable heat shield 892. When the heat shield is removed to enlarge the channel, the amount of heat released by the thermal-energy storage may increase accordingly. In other embodiments, the heat exchange channel can also be heat exchange tubes with valves, solenoid controlled metal thermal switches, and the like.

For the sake of simplicity, only a container 832 of the first circulating system is shown in FIG. 15. Detailed illustrations for the rest may refer to the aforementioned description herein. The container 832 may enclose the thermal-energy storage. The first working fluid enters from an inlet 8321 and the first outcome flows from an outlet 8322. Since the heat storage material used in the thermal-energy storage is generally expensive and dangerous, it is enclosed in the thermal-energy storage and heat exchanged with the working fluid of the first circulating system in the container 832 to achieve the use of heat in this embodiment. Preferably, a thermoelectric conversion member 893 may further be provided on the heat exchange channel between the thermal-energy storage and the container for fully utilizing thermal energy to generate electricity. For example, thermoelectric conversion members can be provided on all the heat exchange channels so as to increase the rate of power generation using thermal energy.

The outlet 8322 can be connected to a steam generator or to a Stirling generator or other thermal energy utilization system depending on demands in use.

When applying the system of the embodiment to a new building n areas with sufficient sunshine, energy self-sufficiency and seasonal energy storage (for example, energy stored in summer can be used in winter) may be achieved for the building.

The principle and implementation manners present disclosure has been described above with reference to specific embodiments, which are merely provided for the purpose of understanding the present disclosure and are not intended to limit the present disclosure.

## Claims

1. An integrated solar energy utilization apparatus, comprising:
at least one photovoltaic conversion member (710) having a front side and a back side;
at least one thermoelectric conversion member (720), wherein the thermoelectric conversion member is a temperature-difference power generation element including at least one first heat-conducting end (721) for inflow of heat and at least one second heat-conducting end (722) for outflow of heat, and when the temperature of the first heat-conducting end (721) is higher than that of the second heat-conducting end (722), the thermoelectric conversion member outputs electric energy; wherein the thermoelectric conversion member (720) is formed as a support of the photovoltaic conversion member (710) with its first heat-conducting end (721) fixed to the back side of the photovoltaic conversion member in a thermally conductive manner; and
a transmission-type Fresnel lens (751) for condensing sunlight to be provided to the front side of the photovoltaic conversion member (710), wherein the transmission-type Fresnel lens (751) has at least one condensing and refracting surface, and the at least one condensing and refracting surface is a tooth surface having at least one Fresnel lens unit,
**characterized by**
a reflective Fresnel lens (752) for condensing sunlight to be provided to the back side of the photovoltaic conversion member (710), wherein the reflective Fresnel lens (752) has at least one condensing and refracting surface, and the at least one condensing and refracting surface is a tooth surface having at least one Fresnel lens unit, and the reflective Fresnel lens (752) is provided with a reflective back side,
wherein the photovoltaic conversion member (710) is a double-sided element capable of absorbing incident sunlight from both of the front and back sides,
wherein the photovoltaic conversion member (710) is arranged between the transmission-type Fresnel lens (751) and the reflective Fresnel lens (752), the thermoelectric conversion member (720) has its second heat-conducting end (722) fixed with respect to the reflective Fresnel lens (752).

2. The apparatus according to claim 1, wherein
the photovoltaic conversion member (710) is connected in a thermally conductive manner to the first heat-conducting end (721) via at least one first heat-conducting member,
the apparatus further comprises a first connection circuit for providing a conductive connection between components at the first heat-conducting end (721) of the thermoelectric conversion member (720), or to provide a conductive connection between the photovoltaic conversion member (710) and the thermoelectric conversion member (720), and
at least one first heat-conducting member is integrated with the first connection circuit on a same substrate which is rigid or flexible.

3. The apparatus according to claim 2, further comprising:
at least one second heat-conducting member for conducting heat from the second heat-conducting end (722), and
a second connection circuit for providing a conductive connection between components at the second heat-conducting end (722) of the thermoelectric conversion member (720),
at least one second heat-conducting member being integrated with the second connection circuit on a same substrate which is rigid or flexible.

4. The apparatus according to claim 3, wherein
the first connection circuit is electrically connected with the second connection circuit, and a plurality of switches are also provided in the whole circuit,
the switches are configured for switching in a current from the photovoltaic conversion member (710) or the outside to input electric energy to the thermoelectric conversion member (720) such that the temperature of the first heat-conducting end (721) decreases and the temperature of the second heat-conducting end (722) increases, or
the switches are configured for circuit switching so that the thermoelectric conversion member (720) outputs electric energy when the temperature of the second heat-conducting end (722) is higher than the temperature of the first heat-conducting end (721), and in this situation, the second heat-conducting end (722) is further configured for inflow of heat and the first heat-conducting end (721) is further configured for outflow of heat, or
the switches are configured for switching in a current from the photovoltaic conversion member (710) or the outside and switching circuit so as to input electric energy to the thermoelectric conversion member (720) such that the temperature of the second heat-conducting end (722) decreases and the temperature of the first heat-conducting end (721) increases.

5. The apparatus according to any one of claims 1 to 4, wherein
there are more than two thermoelectric conversion members (720) which are connected in series or in parallel with the photovoltaic conversion member (710) in a thermally conductive manner.

## Patentansprüche

1. Integrierte Vorrichtung zur Nutzung von Sonnenenergie, umfassend:
mindestens ein photovoltaisches Umwandlungselement (710) mit einer Vorderseite und einer Rückseite;
mindestens ein thermoelektrisches Umwandlungselement (720), wobei das thermoelektrische Umwandlungselement ein Temperaturdifferenz-Energieerzeugungselement ist, das mindestens ein erstes wärmeleitendes Ende (721) zum Zufließen von Wärme und mindestens ein zweites wärmeleitendes Ende (722) zum Abfließen von Wärme enthält, und wenn die Temperatur des ersten wärmeleitenden Endes (721) höher als die des zweiten wärmeleitenden Endes (722) ist, das thermoelektrische Umwandlungselement elektrische Energie abgibt; wobei das thermoelektrische Umwandlungselement (720) als ein Träger des photovoltaischen Umwandlungselements (710) ausgebildet ist, wobei sein erstes wärmeleitendes Ende (721) an der Rückseite des photovoltaischen Umwandlungselements in einer thermisch leitenden Weise befestigt ist;
und
eine Fresnel-Linse (751) vom Transmissionstyp zum Kondensieren von Sonnenlicht, das der Vorderseite des photovoltaischen Umwandlungselements (710) zugeführt wird, wobei die Fresnel-Linse (751) vom Transmissionstyp mindestens eine kondensierende und brechende Fläche aufweist, und die mindestens eine kondensierende und brechende Fläche eine gezahnte Fläche mit mindestens einer Fresnel-Linseneinheit ist,
**gekennzeichnet durch**
eine reflektierende Fresnel-Linse (752) zum Kondensieren von Sonnenlicht, das der Rückseite des photovoltaischen Umwandlungselements (710) zugeführt wird, wobei die reflektierende Fresnel-Linse (752) mindestens eine kondensierende und brechende Fläche aufweist, und die mindestens eine kondensierende und brechende Fläche eine gezahnte Fläche mit mindestens einer Fresnel-Linseneinheit ist, und die reflektierende Fresnel-Linse (752) mit einer reflektierenden Rückseite versehen ist,
wobei das photovoltaische Umwandlungselement (710) ein doppelseitiges Element ist, das einfallendes Sonnenlicht sowohl von der Vorder- als auch von der Rückseite absorbieren kann,
wobei das photovoltaische Umwandlungselement (710) zwischen der Fresnel-Linse (751) vom Transmissionstyp und der reflektierenden Fresnel-Linse (752) angeordnet ist, das zweite wärmeleitende Ende (722) des thermoelektrischen Umwandlungselements (720) in Bezug auf die reflektierende Fresnel-Linse (752) befestigt ist.

2. Vorrichtung nach Anspruch 1, wobei
das photovoltaische Umwandlungselement (710) mit dem ersten wärmeleitenden Ende (721) über mindestens ein erstes wärmeleitendes Element in thermisch leitender Weise verbunden ist,
die Vorrichtung ferner eine erste Verbindungsschaltung umfasst, um eine leitende Verbindung zwischen Komponenten am ersten wärmeleitenden Ende (721) des thermoelektrischen Umwandlungselements (720) bereitzustellen oder um eine leitende Verbindung zwischen dem photovoltaischen Umwandlungselement (710) und dem thermoelektrischen Umwandlungselement (720) bereitzustellen, und
mindestens ein erstes wärmeleitendes Element mit der ersten Verbindungsschaltung auf einem gleichen Substrat integriert ist, das starr oder flexibel ist.

3. Vorrichtung nach Anspruch 2, ferner umfassend:
mindestens ein zweites wärmeleitendes Element zum Leiten von Wärme vom zweiten wärmeleitenden Ende (722), und
eine zweite Verbindungsschaltung, um eine leitende Verbindung zwischen Komponenten am zweiten wärmeleitenden Ende (722) des thermoelektrischen Umwandlungselements (720) bereitzustellen,
wobei mindestens ein zweites wärmeleitendes Element mit der zweiten Verbindungsschaltung auf einem gleichen Substrat integriert ist, das starr oder flexibel ist.

4. Vorrichtung nach Anspruch 3, wobei
die erste Verbindungsschaltung elektrisch mit der zweiten Verbindungsschaltung verbunden ist, und in der gesamten Schaltung auch eine Vielzahl von Schaltern vorgesehen ist,
die Schalter so konfiguriert sind, dass sie einen elektrischen Strom von dem photovoltaischen Umwandlungselement (710) oder von außen einschalten, um dem thermoelektrischen Umwandlungselement (720) elektrische Energie zuzuführen, so dass die Temperatur des ersten wärmeleitenden Endes (721) sinkt und die Temperatur des zweiten wärmeleitenden Endes (722) steigt, oder
die Schalter zum Schalten des Schaltkreises konfiguriert sind, so dass das thermoelektrische Umwandlungselement (720) elektrische Energie ausgibt, wenn die Temperatur des zweiten wärmeleitenden Endes (722) höher als die Temperatur des ersten wärmeleitenden Endes (721) ist, und in dieser Situation das zweite wärmeleitende Ende (722) weiterhin zum Zufließen von Wärme konfiguriert ist und das erste wärmeleitende Ende (721) weiterhin zum Abfließen von Wärme konfiguriert ist, oder
die Schalter konfiguriert sind, um einen elektrischen Strom von dem photovoltaischen Umwandlungselement (710) oder von außen einzuschalten,
und zum Schalten des Schaltkreises konfiguriert sind, um dem thermoelektrischen Umwandlungselement (720) elektrische Energie zuzuführen, so dass die Temperatur des zweiten wärmeleitenden Endes (721) sinkt und die Temperatur des ersten wärmeleitenden Endes (722) steigt.

5. Vorrichtung nach einem der Ansprüche 1 bis 4, wobei mehr als zwei thermoelektrische Umwandlungselemente (720) vorgesehen sind, die mit dem photovoltaischen Umwandlungselement (710) in thermisch leitender Weise in Reihe oder parallel geschaltet sind.

## Revendications

1. Appareil intégré d'utilisation de l'énergie solaire, comprenant :
au moins un élément de conversion photovoltaïque (710) présentant une face avant et une face arrière ;
au moins un élément de conversion thermoélectrique (720), dans lequel l'élément de conversion thermoélectrique est un élément de génération d'énergie par différence de température incluant au moins une première extrémité thermoconductrice (721) pour l'entrée de chaleur et au moins une seconde extrémité thermoconductrice (722) pour la sortie de chaleur, et lorsque la température de la première extrémité thermoconductrice (721) est supérieure à celle de la seconde extrémité thermoconductrice (722), l'élément de conversion thermoélectrique délivre de l'énergie électrique ; dans lequel l'élément de conversion thermoélectrique (720) est formé comme un support de l'élément de conversion photovoltaïque (710) avec sa première extrémité thermoconductrice (721) fixée à la face arrière de l'élément de conversion photovoltaïque d'une manière thermiquement conductrice ; et
une lentille de Fresnel du type à transmission (751) pour condenser la lumière solaire à fournir à la face avant de l'élément de conversion photovoltaïque (710), dans lequel la lentille de Fresnel du type à transmission (751) présente au moins une surface de condensation et de réfraction, et ladite au moins une surface de condensation et de réfraction est une surface de dent comportant au moins une unité de lentille de Fresnel,
**caractérisé par**
une lentille de Fresnel réfléchissante (752) pour condenser la lumière solaire à fournir à la face arrière de l'élément de conversion photovoltaïque (710), dans lequel la lentille de Fresnel réfléchissante (752) présente au moins une surface de condensation et de réfraction, et ladite au moins une surface de condensation et de réfraction est une surface de dent comportant au moins une unité de lentille de Fresnel, et la lentille de Fresnel réfléchissante (752) est pourvue d'une face arrière réfléchissante,
dans lequel l'élément de conversion photovoltaïque (710) est un élément à double face capable d'absorber la lumière solaire incidente à la fois par la face avant et la face arrière,
dans lequel l'élément de conversion photovoltaïque (710) est disposé entre la lentille de Fresnel du type à transmission (751) et la lentille de Fresnel réfléchissante (752), la seconde extrémité thermoconductrice (722) de l'élément de conversion thermoélectrique (720) étant fixée par rapport à la lentille de Fresnel réfléchissante (752).

2. Appareil selon la revendication 1, dans lequel
l'élément de conversion photovoltaïque (710) est connecté de manière thermiquement conductrice à la première extrémité thermoconductrice (721) via au moins un premier élément thermoconducteur,
l'appareil comprend en outre un premier circuit de connexion pour fournir une connexion conductrice entre les composants au niveau de la première extrémité thermoconductrice (721) de l'élément de conversion thermoélectrique (720) ou pour fournir une connexion conductrice entre l'élément de conversion photovoltaïque (710) et l'élément de conversion thermoélectrique (720), et
au moins un premier élément thermoconducteur est intégré au premier circuit de connexion sur un même substrat qui est rigide ou flexible.

3. Appareil selon la revendication 2, comprenant en outre :
au moins un second élément thermoconducteur pour conduire la chaleur depuis la seconde extrémité thermoconductrice (722), et
un second circuit de connexion pour fournir une connexion conductrice entre les composants au niveau de la seconde extrémité thermoconductrice (722) de l'élément de conversion thermoélectrique (720),
au moins un second élément thermoconducteur étant intégré au second circuit de connexion sur un même substrat qui est rigide ou flexible.

4. Appareil selon la revendication 3, dans lequel
le premier circuit de connexion est connecté électriquement au second circuit de connexion et une pluralité de commutateurs sont également prévus dans l'ensemble du circuit,
les commutateurs sont configurés pour commuter un courant provenant de l'élément de conversion photovoltaïque (710) ou de l'extérieur pour entrer de l'énergie électrique dans l'élément de conversion thermoélectrique (720) de telle sorte que la température de la première extrémité thermoconductrice (721) diminue et la température de la seconde extrémité thermoconductrice (722) augmente, ou
les commutateurs sont configurés pour une commutation de circuit de telle sorte que l'élément de conversion thermoélectrique (720) délivre de l'énergie électrique lorsque la température de la seconde extrémité thermoconductrice (722) est supérieure à la température de la première extrémité thermoconductrice (721) et, dans cette situation, la seconde extrémité thermoconductrice (722) est en outre configurée pour une entrée de chaleur et la première extrémité thermoconductrice (721) est en outre configurée pour une sortie de chaleur, ou
les commutateurs sont configurés pour commuter un courant provenant de l'élément de conversion photovoltaïque (710) ou de l'extérieur et pour commuter un circuit de manière à entrer de l'énergie électrique dans l'élément de conversion thermoélectrique (720) de telle sorte que la température de la seconde extrémité thermoconductrice (722) diminue et la température de la première extrémité thermoconductrice (721) augmente.

5. Appareil selon l'une quelconque des revendications 1 à 4, dans lequel plus de deux éléments de conversion thermoélectrique (720) sont connectés en série ou en parallèle avec l'élément de conversion photovoltaïque (710) d'une manière thermiquement conductrice.
